# EUROPEAN PATENT SPECIFICATION

(11) **EP 2 051 699 B1**
(45) Date of publication and mention of the grant of the patent: **26.09.2018**
(21) Application number: 07776843.0
(22) Date of filing: 08.05.2007
(51) Int. Cl.: A61K 9/127, A61K 41/00, A61K 9/00

(54) **PHOTOSENSITIZER FORMULATIONS FOR TOPICAL APPLICATIONS**
PHOTOSENSIBILISATORFORMULIERUNGEN FÜR TOPISCHE ANWENDUNGEN
PRÉPARATIONS DE PHOTOSENSIBILISATEURS POUR APPLICATION TOPIQUE

(30) Priority: 10.05.2006 US 799373 P; 04.05.2007 US 800147
(43) Date of publication of application: 29.04.2009
(73) Proprietor: biolitec Unternehmensbeteiligungs II AG, 1030 Vienna (AT)
(72) Inventor: FARMER, Gerard, 4142 Muenchenstein (CH); SCHEGLMANN, Dietrich, 07751 Jena (DE); ALBRECHT, Volker, 07749 Jena (DE); NIFANTIEV, Nikolay, E., Moscow B-593, 117593 (RU)
(74) Representative: Herrmann, Franz
(86) International application number: PCT/US2007/011045
(87) International publication number: WO 2007/133514

(56) References cited:
- US-A- 5 616 602
- US-A1- 2002 061 330
- US-A1- 2003 064 948
- US-A1- 2004 126 415
- US-A1- 2005 048 109
- BLUME A ET AL: "Interaction of phospholipid liposomes with lipid model mixtures for stratum corneum lipids", INTERNATIONAL JOURNAL OF PHARMACEUTICS, ELSEVIER BV, NL, vol. 99, no. 2-3, 15 October 1993 (1993-10-15), pages 219-228, XP025568572, ISSN: 0378-5173, DOI: 10.1016/0378-5173(93)90364-L [retrieved on 1993-10-15]
- BOIX A ET AL: "Estimation of Transdermal Permeation Parameters in Non-stationary Diffusion Experiments. Application to Pre-treatment Studies with Terpenes", PHARMACEUTICAL RESEARCH, KLUWER ACADEMIC PUBLISHERS-PLENUM PUBLISHERS, NL, vol. 22, no. 1, 1 January 2005 (2005-01-01), pages 94-102, XP019370696, ISSN: 1573-904X, DOI: 10.1007/S11095-004-9014-2

## Description

### 3. Invention Disclosure Statement

Skin has been used for decades as a site for topical administration of drug for treating dermatological problems, for cosmetic application and for treating cancerous and precancerous conditions in skin. The main aim of a topical drug delivery system is to maximize the delivery of therapeutic agent in the formulation through stratum corneum, into deeper layers of the skin. Other aims are to promote uniform absorption, to offer controlled delivery of the therapeutic agents and to reduce side effects.

The skin is a complex membrane composed of different layers. Its upper layer, stratum corneum, is the main physical barrier for percutaneous penetration of drug applied topically. There are several different pharmaceutical products which are targeted through the skin into the body and these include transdermal drug delivery systems (patches), liposomes, creams, ointments, lotions, gels as well as subcutaneous implants. In contrast to the traditional oral drug administration, the use of transdermal drug delivery by-passes first pass metabolism of the liver, the acidic environment of the gastrointestinal tract, and problems of absorption in the stomach etc.

In topical administration, penetration rate is influenced by many factors like molecular weight of the drug, drug carrier, interaction of carrier with the skin and many others. The permeability of the drug molecule is directly related to its lipophilicity and inversely proportional to molecular size. Attempts have been made to overcome this limitation. Different drug delivery systems are described in the prior art to overcome the skin barrier.

Liposomes have been much investigated as a system for dermal application. But most studies have shown that these liposome vesicles are not very efficient in penetrating the physical skin barrier as these conventional liposome vesicles arc rigid. Specialized liposomal which are flexible to pass through the skin are being explored. Transfersomes in one such flexible liposomes example described by Gregor Ccve in US patent number 6,165,500 for systemic administration of insulin. Transfersomes are lipid vesicles that incorporate molecular edge activators, which can deform and squeeze through microspores in stratum corneum. Similarly niosomes and ethosomes arc other carrier used for dermal application of drug. Ethosomes are liposomes high in ethanol content which fluidizes the ethosomal lipids and stratum corneum bilayer thus allowing the penetration; while niosomes uses nonionic surfactant.

Many of the above described drug carrier systems are not efficient enough to transport the photosensitizer across the skin barrier, as most of them face a lot of stability related problems e.g. deformation of the liposome vesicle to pass through the skin pore can cause damage to carrier system leading to leakage of the active compound carried in the liposome. Other problems are liposome vesicles can aggregate on passing the skin barrier reducing their efficiency and ability to bring the photo drug to the desired treatment site.

The success of this drug delivery system depends on the ability of the drug to penetrate through the skin in sufficient quantities to achieve its desired therapeutic effect. It has been shown that fluidization of intercellular domains and thus altering the stratum corneum is a possible mechanism for enhanced transport of drug encapsulated vesicles.

Different methods are used in the prior art to modify the barrier properties of the stratum corneum and to enhance drug penetration through skin which include use of chemical penetration enhancers; iontophoresis, which is administrating ionic therapeutic agents through skin using a low level of electric current; and, phonophoresis which uses ultrasound energy to increase penetration through skin.

Photodynamic therapy (PDT) is one of the most promising new techniques being explored for use in a variety of medical applications and is known as a well-recognized treatment for the destruction of tumors. The use of PDT for the treatment of various types of disease is limited due to the inherent features of photosensitizers. These include their high cost, extended retention in the host organism, skin photo toxicity, low solubility in physiological solutions (which reduces its usefulness for intravascular administration as it can provoke thromboembolic accidents), and low targeting effectiveness. These disadvantages generally lead to the administration of very high doses of a photosensitizer, which dramatically increase the possibility of accumulation of the photosensitizer in non-damaged tissues and the accompanying risks of affecting non-damaged sites.

The document US2005048109 discloses liposomes encapsulating temoporfin for injection. Since the application of photodynamic therapy is used in treating many dermatological problems and also in cancerous and precancerous skin conditions it is necessary to develop an effective drug delivery system which can be applied topically. So as to optimize and target the abnormal cells in the skin and beneath, by topical application thus reducing the side effects like skin photosensitivity in the other normal skin cells.

Drug delivery systems designed in the present invention are better than the prior art systems. These delivery systems can effectively penetrate the skin barrier and optimize the drug concentration in deeper layer of the skin.

Present invention aims at overcoming the impermeability of intact human skin to penetration in a reversible and non-damaging manner as well as to design therapeutically effective intradermal drug delivery systems for transporting hydrophobic photosensitizers for administering photodynamic therapy.

### Objectives and Brief Summary of the Invention

It is an objective of the present invention to formulate a suitable highly flexible, penetrating liposomal carrier system for topical application.

It is another objective of the present invention to use this delivery system for faster and efficient transport of hydrophobic photosensitizer across stratum corneum.

It is a further objective of the present invention to use the new formulation for optimizing and targeting photosensitizer through topical administration to deeper layers within the skin.

It is yet another objective of this invention to increase the drug penetration across the skin barrier as well as decreasing the side effects on the skin.

Briefly stated, the present invention provides a highly flexible penetrating liposomal carrier system, formulated with enhanced skin penetration properties. This specialized formulation of a highly flexible penetrating liposomal delivery system for use in topical applications of photodynamic therapy is defined in the claims. This new formulation can squeeze the liposomal particles through intercellular regions of stratum corneum as an intact structures, and in this way, deliver encapsulated photosensitizer to the epidermis, dermis, hypodermis and surroundings. The penetrating, liposomal formulations provide therapeutically effective amounts of a hydrophobic photosensitizer temoporfin (mTHPC) through topical application with better skin penetration thus improving drug targeting and the efficacy of photodynamic therapy (PDT).

### Brief Description of the Drawings

**Figure 1** is a gel filtration curve of highly flexible penetrating liposomal formulated temoporfin, chemical name m-tetrahydroxyphenylchlorin (mTHPC). Both, lipid components and mTHPC show the same distribution over all fractions collected.
**Figure 2** illustrates the mTHPC wet weight concentration in blood, liver, muscle, skin, skin treated, spleen and tumor after 0, 0.5, 3 and 6 hours of topical application of highly flexible penetrating liposomal formulation loaded with mTHPC on left thigh of the mice.
**Figure 3** depicts the mTHPC wet weight concentration in blood, liver, muscle, skin, skin treated, spleen and tumor at different time intervals of the application of a highly flexible penetrating liposomal formulation loaded with mTHPC on the tumor of the mice.
**Figure 4** represents the percentage of injected dose of mTHPC in different tissues at different intervals of topical application of a highly flexible penetrating liposomal formulation of mTHPC on left thigh of the mice.
**Figure 5** shows the percentage of injected dose of mTHPC in different tissues at different time interval of topical application of a penetrating liposomal formulation of mTHPC directly on the tumor of the mice.

### Detailed Description of the Preferred Embodiments

A penetrating liposomal formulation is a specialized topical drug delivery system that can reach cells in the deeper skin layer but avoids systemic absorption of the drug thus minimizing side effects. Highly specialized penetrating liposomal formulation of present invention comprises phospholipid mixture that is 73-76% by weight (3-phosphatidyl)choline, maximum 6% by weight (3-lysophosphatidyl)choline, maximum 7% by weight cephalin and maximum 7% by weight phosphatidic acid and a therapeutically effective amount of a hydrophobic photosensitizer, temoporfin (mTHPC). These new formulations are highly specialized flexible liposomes which can easily penetrate through stratum corneum and reach the deeper cells of the skin layer even when they are topically applied. This allows the drug to be delivered without significant side effects which are commonly reported with systemic administration.

Topical applications of dermatological drugs can now be used to achieve a localized therapeutic effect on or beneath the application site. The principal goals of topical drug delivery are to maximize the delivery of the drug or active ingredient from the formulation through the stratum corneum, upper epidermis, and into deeper subcutaneous tissue, to minimize the further absorption through the skin into the systemic circulation, and further to promote a more uniform absorption, to reduce dose frequency and to improve the cosmetic or aesthetic appeal of the dosage form.

As with other routes of administration, transport across the skin is also associated with several disadvantages, the main being, not all drugs arc suitable candidates for topical administration. A number of physicochemical parameters have been identified to influence drug uptake by skin such as size of the vesicles, molecular weight that influences the diffusion process, and variations in penetration rates in different skin types. In other words the skin permeability of the molecule is directly proportional to its lipophilicity and inversely proportional to molecular size. Sometime it is seen that damaged stratum corneum shows increased permeability compared to intact skin layer. Hyperproliferation of stratum corneum was found to increase permeability due to reduced barrier properties.

The new penetrating liposomal formulation of this invention overcomes most of the drawbacks seen in the convention delivery systems. These new formulations show enhanced shelf life, non-aggregated and stable with better load carrying capacity. The penetrating liposome vesicle is formulated at nano scale to help easy transport through the skin barrier, since particle size is an important criterion for transdermal penetration. These newly formulated penetrating liposome particles can squeeze themselves through intercellular regions of stratum corneum as intact structures, and by this way, deliver encapsulated photosensitizer to the dermis.

The present invention is further illustrated by the following examples, but is not limited thereby.

### EXAMPLE 1 - Preparation of highly flexible penetrating liposomal vesicles containing meta-tetrahydroxyphenylchlorin (mTHPC)

A specialized penetrating liposome formulation of mTHPC also called Temoporfin was prepared according to the following general procedure:
Ethanolic solution of lipids (3:1) is vigorously mixed with a vortexer in a sealed glass vessel for 5 minute. Then m-THPC and penetration enhancer were added and the mixture was again mixed. This was followed by ultrasonication for 5 minute. A suitable amount of phosphate buffer saline pH 7.4 was added drop wise to hydrate the mixture and is mixed again with a vortexer for 5 minute followed by ultrasonication. The resultant mixture is then extruded (filtrated) through different polycarbonate filtrate with pore sizes form 400 nm up to 50 nm. The entire process is performed at room temperature.

Using the foregoing procedure, a highly penetrating liposomal formulation for m-THPC was prepared as follows:

| **Ingredient** | **Amount %w/v** |
|---|---|
| mTHPC | 0.2 to 1.5 mg/ml |
| Phospholipon® 80 | 10 |
| Ethanol | 3 |
| Na2HPO4 | 2mg/ml |
| KH2PO4 | 0.2mg/ml |
| Cineol | 0.9 |
| Citral | 0.9 |
| Limonene | 0.2 |
| Water | as necessary |

The Phospholipon ® 80 is a commercially available lipid mixture comprising of about 73.0-76.0% by weight (3-sn-phosphatidyl) choline, maximum 6% by weight (3-sn-lysophosphatidyl) choline, maximum. 7% by weight cephalin and maximum 7% by weight phosphatidic acid. (Phospholipon ® 80 used in the preparation of penetrating liposomes in the present invention; is supplied by Phospholipid GmbH; Nattermannallee 1; D-50829 Köln, Germany).

The following commercially available lipid mixtures are not in the scope of the claims: Phospholipon 50 (comprising of about min. 50% by weight 3-Sn-phosphatidyl choline and max. 6% by weight 3-Sn-Lysophosphatidyl choline), Phospholipon 90 (comprising of minimum about 90% by weight 3-Sn-Phosphatidyl choline, max. 6% by weight 3-Sn-lysophosphatidyl choline and max. 3% by weight tocopherol) and Phospholipon 100.

Penetration enhancer is used to increase skin permeability by reversibly damaging the physiochemical nature of the stratum corneum to reduce its diffusional resistance. A good skin penetration enhancer should be non-irritating, pharmacological inert, non-toxic, and should be easy to be used with other ingredients of the formulation. In the above formulations, terpenes such as Cineol (2,2,4-trimethyl-3-oxabicyclo[2.2.2]octane), Citral (3,7-dimethyl-2,6-octadienal) and Limonen (1-methyl-4-pro-1-en-2-yl-cyclohexene) are used as penetration enhancers to improve the absorption of highly penetrating liposomal formulated mTHPC through the stratum corneum barrier of the skin. Many terpenes have been classified as GRAS (Generally Regarded As Safe) by FDA. Penetration enhancer can help the new penetrating liposomal formulation to easily pass through main skin barrier by increasing the fluidity of stratum corneum, thereby reducing its resistance. Phospholipids used in the new liposomal formulation of the present invention show better deformability to the vesicles resulting in better skin permeation.

### Stability data

### a. Standard highly flexible penetrating liposome without mTHPC (stored at 4°C)for 0 day, 3, 6, and 12 months.

| **Times of measurement** | **effective Ø (nm)** |
|---|---|
| 0 day | 139nm |
| ∼3month | 172nm |
| ∼6month | 187nm |
| ∼12 months | 170nm |

### b. Stability data for highly flexible penetrating liposome with different concentration of mTHPC prepared by ultra turrax method.

| **mTHPC (mg/ml) (stored at 4°C)** | **0.75** | **01.5** | **2.5** | **4.0** |
|---|---|---|---|---|
| **Effective Ø (nm) for** | | | | |
| 0 day | 155 | 142 | 212 | 223 |
| ∼3 months | 187 | 168 | 481 | 414 |
| ∼6 months | 202 | 177 | 497 | 580 |
| ∼12 months | 186 | 163 | 506 | Not available. |

### c. Stability for penetrating liposome with 0.75mgTHPC/ml content.

### Example 1 (storage temperature 4°C)

| **Times of measurement** | **effective Ø (nm)** |
|---|---|
| ∼3 Months | 105 |
| ∼6 months | 117 |
| ∼12 months | 115 |

### Example 2 (storage temperature 4°C)

| **Times of measurement** | **effective Ø (nm)** |
|---|---|
| ∼3 Months | 107 |
| ∼6 months | 115 |
| ∼12 months | 102 |

The above data shows a penetrating liposomal mTHPC formulation which is stable.

### Stability data for penetrating liposomal formulation with different lipids

| Lipids | | mTHPC (mg/ml) | Eff. Ø **(nm)** |
|---|---|---|---|
| Ph 80 in Isopropanol | 0 day | 0.75 | 118 |
| | 200 days | | 135 |
| | 312 days | | 127 |
| Ph 50 in propylene glycol | 0 day | 0.75 | 196 |
| | 200 days | | 499 |
| | 312 days | | 585 |

The above stability data shows that the much preferred lipid for formulation is Ph 80 in isopropanol compared to Ph 50 in propylene glycol in regard to long-term shelf life. For applications where much shorter shelf lives are appropriate the Ph 50 system can also be used. The effective diameter of the liposome produced from Ph 80 in Isopropanol over a period of time shows better stability compared to the Ph50 in propylene glycol. Thus Ph 80 is a preferred embodiment over the Ph 50 in preparing liposomes for most applications.

### Filtration behavior of highly flexible penetrating liposomes

Diluted solutions of this penetrating liposomes with mTHPC where measured at two different wavelengths to estimate the percentage of drug (mTHPC) lost during the filtration through 0.2µm and 0.02µm filters. Penetrating liposomes were able to cross the 0.02 µm membrane system without substantial loss of drug, whereas conventional liposomes are not. This shows the high flexibility of the liposome as they are able to cross membranes with a pore size of less than 20% of their own diameter. The difference of the mTHPC content at two different wavelengths (649 and 703nm) was calculated to show that there no net loss of mTHPC content was observed between before and after filtration. This demonstrates a high efficiency of encapsulation of the drug.

### Localization of mTHPC within the specialized penetrating liposomal formulation

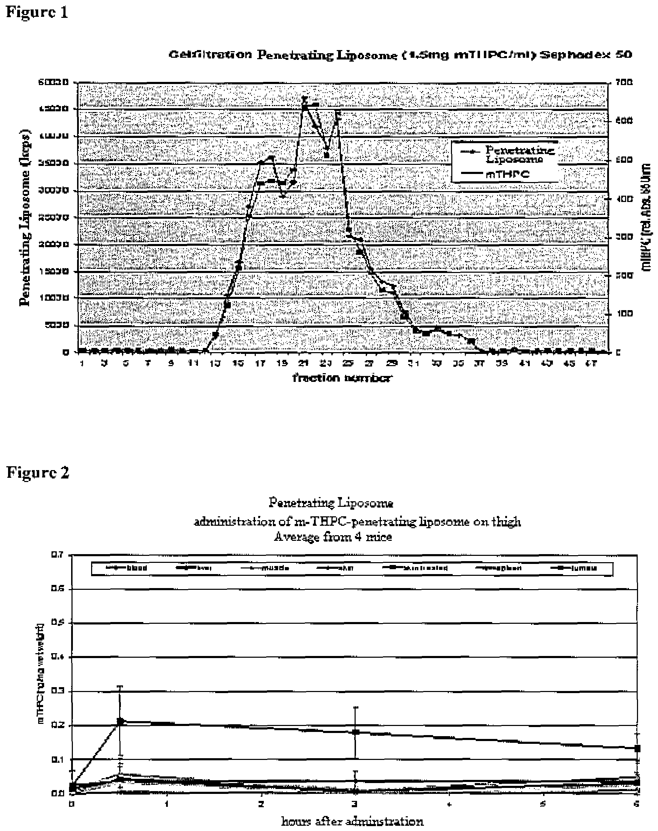

Gel filtration of highly specialized liposomal formulation performed on Sephadex G50 columns. As shown in Figure 1, lipids and mTHPC show the same distribution over all fractions indicating a physically interaction of both components i.e. integration of mTHPC into the membrane bilayer, demonstrating high encapsulation efficiency and stability.

### Animal study results

In vivo studies were performed in female NMRI mice of 6 to 8 week old, inoculated with HT29 human colorectal carcinoma cell. Of the three treatment groups, 1st group served as a control while 2^{nd} and 3^{rd} group received the new penetrating liposomal formulated mTHPC on left thigh and tumor respectively. In the treatment groups the mTHPC content is checked at different time periods to study the effectiveness of the new penetrating liposomal formulation in transferring drug through the skin barrier to the targeted region in deeper layers of the skin. Four mice for each treatment group were sacrificed to record the mTHPC content in blood, skin, treated skin, tumor, muscle, liver and spleen at 0, 0.5, 3 and 6 hours after topical application of the new penetrating liposomal formulated mTHPC.

Figure 2 shows the mTHPC wet weight concentration in blood, liver, muscle, skin, skin treated, spleen and tumor after 0, 0.5, 3 and 6 hours of topical administration of penetrating liposomal with mTHPC in left thigh of the mice. The highest concentration of mTHPC in the tumor is reached with the 0.5 hours of topical administration thus reducing the drug light interval. The mTHPC concentration in other tissues is cleared soon.

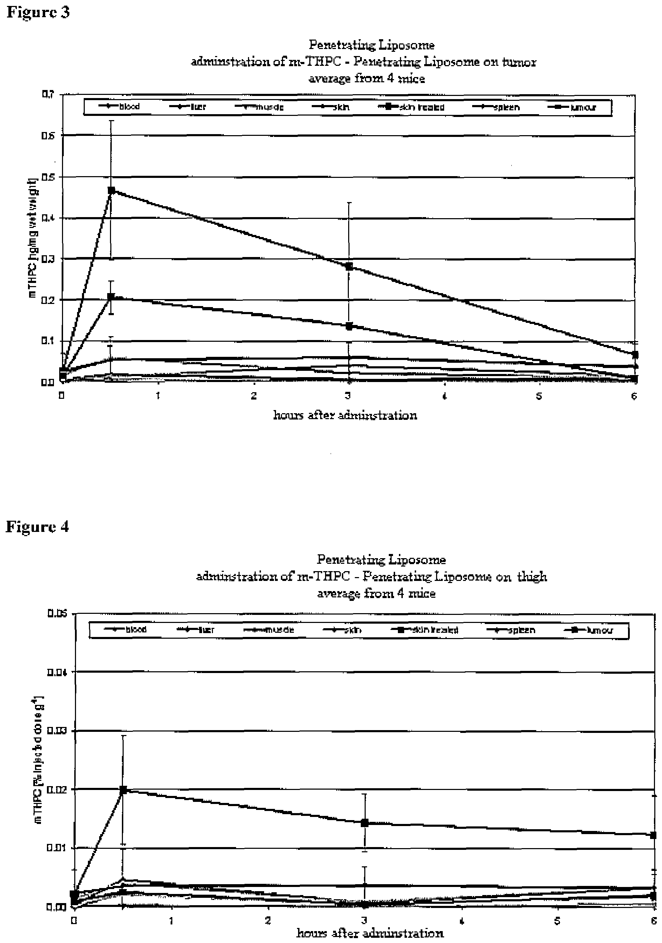

Second group of mice were treated with penetrating liposomal formulation loaded with mTHPC, which, was applied topical on the tumor and the mTHPC wet weight concentration in blood, liver, muscle, skin, skin treated, spleen and tumor at different time interval is plotted in Figure 3. The maximum mTHPC concentration in tumor is reached 0.5 hours after topical application of new liposomal formulation.

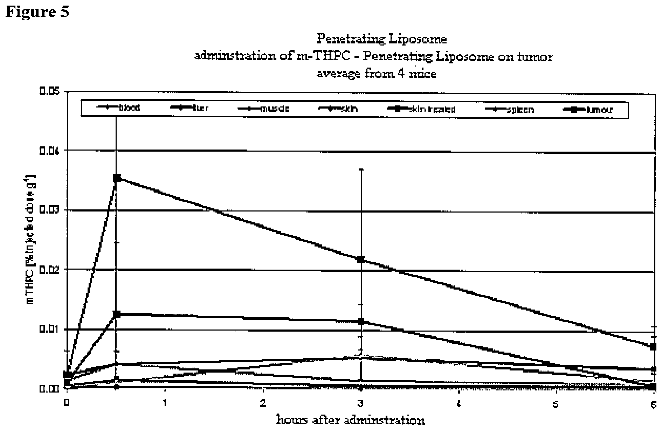

Figure 4 shows the percentage of injected dose of mTHPC in different tissues after 0, 0.5, 3 and 6 hours of topical administration of new liposomal formulation on left thigh of the mice. Figure 5 illustrates the percentage of injected dose of mTHPC in different tissues after 0, 0.5, 3 and 6 hours of topical administration of penetrating liposomal formulated mTHPC on the tumor of the mice. Highest tumor concentration for both was observed after 0.5 hours of topical application on the tumor.

In all the above studies the mTHPC concentration in other tissues especially liver and spleen was lowered considerably thus reducing the toxicity to these critical tissues in the body.

## Claims

1. A pharmaceutical liposomal formulation for use in topical applications of photodynamic therapy, with enhanced penetration stability and delivery, comprising;
a highly flexible, penetrating liposomal carrier system, wherein said carrier system is a liposomal bilayer comprising phospholipids and wherein said phospholipids are a mixture consisting of 73-76% by weight (3-phosphatidyl) choline, maximum 6% by weight (3-lysophosphatidyl) choline, maximum 7% by weight cephalin and maximum 7% by weight phosphatidic acid;
a therapeutically effective amount of a hydrophobic photosensitizer, contained in said liposomal bilayer wherein said hydrophobic photosensitizer is temoporfin (mTHPC);
additional skin penetration enhancers; and
wherein said highly flexible penetrating liposomal carrier system can penetrate through an intact stratum corneum or wound surface without premature removal of said hydrophobic photosensitizer from said bilayer and without agglomeration of said liposomal bilayers as they pass through said stratum corneum or wound surface.

2. The pharmaceutical liposomal formulation for use in topical applications of photodynamic therapy according to claim 1, wherein said skin penetration enhancers are terpenes and terpenoids selected from the group consisting of 2,2,4-trimethyl-3-oxabicyclo[2,2,2]octane, 3,7-dimethyl-2,6-octadienal, and 1-methyl-4-pro-1-en-2yl-cyclohexene.

3. The pharmaceutical liposomal formulation for use in topical applications of photodynamic therapy according to claim 1 or 2, wherein said therapeutically effective concentration of photosensitizer is from 0.0001 to 0.4 percent w/v.

## Patentansprüche

1. Eine pharmazeutische liposomale Formulierung für die Verwendung bei topischen Anwendungen der photodynamischen Therapie mit verbesserter Eindringstabilität und Zuführung mit:
einem hochflexiblen eindringenden liposomalen Trägersystem, wobei das Trägersystem eine liposomale Doppelschicht ist, die Phospholipide aufweist und wobei die Phospholipide eine Mischung sind, die aus 73-76 Gew.-% (3-Phosphatidyl)-cholin, maximal 6 Gew.-% (3-Lysophosphatidyl)-cholin, maximal 7 Gew.-% Cephalin und maximal 7 Gew.-% Phosphatidsäure besteht;
einer therapeutisch wirksamen Menge eines hydrophoben Photosensibilisators, der in der liposomalen Doppelschicht enthalten ist, wobei der hydrophobe Photosensibilisator Temoporfin (mTHPC) ist;
zusätzliche Fördermittel für das Eindringen in die Haut, und
wobei das hochflexible eindringende liposomale Trägersystem durch ein intaktes Stratum corneum oder Wundoberfläche eindringen kann, ohne vorzeitige Entfernung des hydrophoben Photosensibilisators aus der Doppelschicht und ohne Zusammenfallen der liposomalen Doppelschichten, wenn diese durch das Stratum corneum oder die Wundoberfläche hindurchtreten.

2. Pharmazeutisch liposomale Formulierung für die Verwendung in topischen Anwendungen der photodynamischen Therapie nach Anspruch 1, bei der die Fördermittel zum Eindringen in die Haut Terpene oder Terpenoide sind, die aus der Gruppe ausgewählt sind, die aus 2,2,4-Trimethyl-3-oxabicyclo[2,2,2]octan, 3,7-Dimethyl-2,6-octadienal, und 1-Methyl-4-pro-1-en-2yl-cyclohexen besteht.

3. Pharmazeutische liposomale Formulierung zur Verwendung in topischen Anwendungen der photodynamischen Therapie nach Anspruch 1 oder 2, bei der die therapeutisch wirksame Konzentration des Photosensiblisiators zwischen 0,0001 bis 0,4 Prozent [m/V] ist.

## Revendications

1. Formulation liposomale pharmaceutique destinée à être utilisée dans des applications topiques de thérapie photodynamique, avec une stabilité de pénétration et un acheminement amplifiés, comprenant :
un système de vecteur liposomal de pénétration hautement flexible, dans laquelle ledit système de vecteur est une bicouche liposomale comprenant des phospholipides et dans laquelle lesdits phospholipides sont un mélange constitué de 73 à 76 % en poids de (3-phosphatidyl) choline, 6 % en poids maximum de (3-lysophosphatidyl) choline, 7 % en poids maximum de céphaline et 7 % en poids maximum d'acide phosphatidique ;
une quantité thérapeutiquement efficace d'un photosensibilisateur hydrophobe contenu dans ladite bicouche liposomale, dans laquelle ledit photosensibilisateur hydrophobe est la témoporfine (mTHPC) ;
des amplificateurs de pénétration cutanée supplémentaires ; et
dans laquelle ledit système de vecteur liposomal de pénétration hautement flexible peut pénétrer à travers une couche cornée intacte ou surface de lésion sans élimination prématurée dudit photosensibilisateur hydrophobe de ladite bicouche et sans agglomération desdites bicouches liposomales alors qu'elles passent à travers ladite couche cornée ou surface de lésion.

2. Formulation liposomale pharmaceutique destinée à être utilisée dans des applications topiques de thérapie photodynamique selon la revendication 1, dans laquelle lesdits amplificateurs de pénétration cutanée sont des terpènes et terpénoïdes sélectionnés parmi le groupe constitué du 2,2,4-triméthyl-3-oxabicyclo[2,2,2]octane, 3,7-diméthyl-2,6-octadiénal et 1-méthyl-4-pro-1-én-2yl-cyclohexène.

3. Formulation liposomale pharmaceutique destinée à être utilisée dans des applications topiques de thérapie photodynamique selon la revendication 1 ou 2, dans laquelle ladite concentration thérapeutiquement efficace en photosensibilisateur va de 0,0001 à 0,4 pourcent en poids/volume.
